# EUROPEAN PATENT APPLICATION

(11) **EP 4 850 660 A1**
(43) Date of publication of application: **09.12.2026**
(21) Application number: 24925746.0
(22) Date of filing: 19.07.2024
(51) Int. Cl.: A61K 8/34, A61K 8/22, A61K 9/08, A61K 33/40, A61K 47/10, A61P 39/06, A61Q 5/00, A61Q 11/00, A61Q 19/00

(54) **ACTIVE OXYGEN SCAVENGER, METHOD FOR ELIMINATING ACTIVE OXYGEN, AND METHOD FOR PRODUCING ACTIVE OXYGEN SCAVENGER**

(30) Priority: 19.02.2024 JP 2024023291
(71) Applicant: Mediplus IP Solutions, Inc., Tokyo 150-0013 (JP)
(72) Inventor: SHIOTA Gotaro, Tokyo 150-0013 (JP); OKUMURA Tatsuya, Tokyo 150-0013 (JP)
(74) Representative: J A Kemp LLP
(86) International application number: PCT/JP2024/026036
(87) International publication number: WO 2025/177592

(57) **Abstract**

The present disclosure provides a novel active oxygen scavenger. The active oxygen scavenger of the present disclosure includes an ozone-treated alcohol ozone-treated with an oxygen allotrope-containing gas.

## Description

### TECHNICAL FIELD

The present disclosure relates to an active oxygen scavenger, an active oxygen scavenging method, and a method for producing an active oxygen scavenger.

### BACKGROUND ART

Active oxygen refers to a state in which oxygen becomes a radical or the like and is activated more than usual. Active oxygen reacts with various components in a metabolic process in a body, and causes cell damage when becoming excessive (Non Patent Literature 1).

### Citation List

### Non Patent Literature

Non Patent Literature 1: https://www.e-healthnet.mhlw.go.jp/information/food/e-04-003.html (Ministry of Health, Labour and Welfare, Health information site for prevention of lifestyle-related diseases, retrieval date: February 15, 2024)

### SUMMARY OF INVENTION

### Technical Problem

For such a reason, the industrial utility value of the active oxygen scavenger is great.

In view of the above, an object of the present disclosure is to provide a novel active oxygen scavenger, active oxygen scavenging method, and method for producing an active oxygen scavenger.

### Solution to Problem

In order to achieve the above object, the present disclosure provides an active oxygen scavenger including an ozone-treated alcohol ozone-treated with an oxygen allotrope-containing gas.

The present disclosure also provides an active oxygen scavenging method including reacting an ozone-treated alcohol ozone-treated with an oxygen allotrope-containing gas with active oxygen.

The present disclosure also provides a method for producing an active oxygen scavenger including ozone-treating an alcohol with an oxygen allotrope-containing gas. Advantageous Effects of Invention

The present disclosure can provide a novel active oxygen scavenger, active oxygen scavenging method, and method for producing an active oxygen scavenger.

### BRIEF DESCRIPTION OF DRAWINGS

[FIG. 1] FIG. 1 is an ESR spectrum diagram of OG200 of Examples.
[FIG. 2] FIG. 2 is an ESR spectrum diagram of OG20 of Examples.
[FIG. 3] FIG. 3 is an ESR spectrum diagram of OG4 of Examples.
[FIG. 4] FIG. 4 is a bar graph showing active oxygen scavenging rates (%) calculated from the ESR spectra of FIGs. 1 to 3.
[FIG. 5] FIG. 5 is an ESR spectrum diagram of synthetic glycerin.
[FIG. 6] FIG. 6 is an ESR spectrum diagram of concentrated glycerin for cosmetics (Kao Corporation).
[FIG. 7] FIG. 7 is an ESR spectrum diagram of reagent special grade glycerin (FUJIFILM Wako Pure Chemical Corporation).
[FIG. 8] FIG. 8 is an ESR spectrum diagram of Japanese Pharmacopoeia concentrated glycerin (Kao Corporation).
[FIG. 9] FIG. 9 is an ESR spectrum diagram of Japanese Pharmacopoeia concentrated glycerin (Sakamoto Yakuhin Kogyo Co., Ltd.).
[FIG. 10] FIG. 10 is a bar graph showing active oxygen scavenging rates (%) calculated from the ESR spectra of FIGs. 5 to 9.
[FIG. 11] FIG. 11 is an ESR spectrum diagram of Example 2 ozone-treated using oxygen (O₂) in an oxygen cylinder as a raw material, in a case of a treatment time of 3 hours.
[FIG. 12] FIG. 12 is an ESR spectrum diagram of Example 2 ozone-treated using oxygen (O₂) in an oxygen cylinder as a raw material, in a case of a treatment time of 6 hours.
[FIG. 13] FIG. 13 is an ESR spectrum diagram of Example 2 ozone-treated using oxygen (O₂) in an oxygen cylinder as a raw material, in a case of a treatment time of 24 hours.
[FIG. 14] FIG. 14 is an ESR spectrum diagram of Example 3 ozone-treated using oxygen (O₂) in air as a raw material, in a case of a treatment time of 3 hours.
[FIG. 15] FIG. 15 is an ESR spectrum diagram of Example 3 ozone-treated using oxygen (O₂) in air as a raw material, in a case of a treatment time of 6 hours.
[FIG. 16] FIG. 16 is an ESR spectrum diagram of Example 3 ozone-treated using oxygen (O₂) in air as a raw material, in a case of a treatment time of 24 hours.
[FIG. 17] FIG. 17 is an ESR spectrum diagram of Example 1 ozone-treated using oxygen concentrated by a PSA (oxygen concentrator) as a raw material, in a case of a treatment time of 3 hours.
[FIG. 18] FIG. 18 is an ESR spectrum diagram of Example 1 ozone-treated using oxygen concentrated by a PSA (oxygen concentrator) as a raw material, in a case of a treatment time of 6 hours.
[FIG. 19] FIG. 19 is an ESR spectrum diagram of Example 1 ozone-treated using oxygen concentrated by a PSA (oxygen concentrator) as a raw material, in a case of a treatment time of 24 hours.
[FIG. 20] FIG. 20 is an ESR spectrum diagram of Example 1 ozone-treated using oxygen concentrated by a PSA (oxygen concentrator) as a raw material, in a case of a treatment time of 96 hours.
[FIG. 21] FIG. 21 is a bar graph showing active oxygen scavenging rates (%) of Example 2 ozone-treated using oxygen (O₂) as a raw material, in cases of treatment times of 3 hours, 6 hours, and 24 hours.
[FIG. 22] FIG. 22 is a bar graph showing active oxygen scavenging rates (%) of Examples 1 to 3 in a case of a treatment time of 24 hours.
[FIG. 23] FIG. 23 is a bar graph showing active oxygen scavenging rates (%) in a case where a 100% glycerin solution, a 50% aqueous glycerin solution, and a 5% aqueous glycerin solution are respectively used, in a case where the ozone treatment time of Example 1 is 24 hours.
[FIG. 24] FIG. 24 is a bar graph showing active oxygen scavenging rates (%) before and after heating an aqueous glycerin solution of Example 1 having an ozone treatment time of 96 hours at 50°C for 2 weeks.
[FIG. 25] FIG. 25 is a bar graph showing active oxygen scavenging rates (%) before and after storing an aqueous glycerin solution of Example 1 having an ozone treatment time of 96 hours at room temperature for 3 to 24 hours.
[FIG. 26] FIG. 26 is a graph showing a correlation between a treatment time and a pH in each of Examples 1 to 3.

### DESCRIPTION OF EMBODIMENTS

In the present specification, in a case where an isomer such as a tautomer or a stereoisomer (e.g., a geometric isomer, a conformer, and an optical isomer) exists in a compound, any isomer can be used in the present disclosure unless otherwise specified. In addition, in the present disclosure, in a case where a substance can form a salt, the salt can also be used in the present disclosure unless otherwise specified. The salt may be an acid addition salt or a base addition salt. Furthermore, the acid forming the acid addition salt may be an inorganic acid or an organic acid, and the base forming the base addition salt may be an inorganic base or an organic base. The inorganic acid is not particularly limited, and examples thereof include sulfuric acid, phosphoric acid, hydrofluoric acid, hydrochloric acid, hydrobromic acid, hydroiodic acid, hypofluorous acid, hypochlorous acid, hypobromous acid, hypoiodous acid, fluorous acid, chlorous acid, bromous acid, iodous acid, fluoric acid, chloric acid, bromic acid, iodic acid, perfluoric acid, perchloric acid, perbromic acid, and periodic acid. The organic acid is also not particularly limited, and examples thereof include p-toluenesulfonic acid, methanesulfonic acid, oxalic acid, p-bromobenzenesulfonic acid, carbonic acid, succinic acid, citric acid, benzoic acid, and acetic acid. The inorganic base is not particularly limited, and examples thereof include ammonium hydroxide, an alkali metal hydroxide, an alkaline earth metal hydroxide, a carbonate, and a hydrogen carbonate. More specifically, examples thereof include sodium hydroxide, potassium hydroxide, potassium carbonate, sodium carbonate, sodium hydrogen carbonate, potassium hydrogen carbonate, calcium hydroxide, and calcium carbonate. The organic base is also not particularly limited, and examples thereof include ethanolamine, triethylamine, and tris(hydroxymethyl)aminomethane. The method for producing these salts is also not particularly limited, and these salts can be produced, for example, by a method such as appropriately adding the acid or the base as described above to the compound by a known method.

Hereinafter, the present disclosure will be described more specifically with reference to examples. However, the present disclosure is not limited by the following description. In addition, descriptions in the present disclosure can be incorporated by reference to each other unless otherwise specified. In the present specification, in a case where the expression "to" is used between values, the expression is used in a meaning including a numerical value or a physical value before and after the expression. In addition, in the present specification, the expression "A and/or B" includes "only A", "only B", and "both A and B".

### [1. Active Oxygen Scavenger and Method for Producing Active Oxygen Scavenger]

The active oxygen scavenger of the present disclosure includes an ozone-treated alcohol ozone-treated with an oxygen allotrope-containing gas, as described above. Specifically, since ozone is generated using an oxygen-containing gas contained in the oxygen allotrope-containing gas as a raw material, the alcohol can be ozone-treated with the ozone. The ozone-treated alcohol is preferably ozone-treated glycerin. The structure of the ozone-treated alcohol is not particularly limited, and the ozone-treated alcohol preferably includes an ozone-treated alcohol having an active oxygen scavenging function. In the present disclosure, the structure of the ozone-treated alcohol having the active oxygen scavenging function is also not particularly limited, and may be any structure as long as it has the active oxygen scavenging function.

In the present disclosure, examples of the oxygen allotrope include oxygen (O₂) and ozone (O₃), and ozone is particularly preferable.

In the present disclosure, the alcohol may be a monohydric alcohol, a polyhydric alcohol, or a sugar alcohol, and a polyhydric alcohol is preferable. In addition, the alcohol may be a saturated alcohol or an unsaturated alcohol, may be linear, may or may not necessarily include a branched chain, may or may not necessarily include a cyclic structure, may be an aliphatic alcohol or an aromatic alcohol, and may be a natural product or a synthetic product. Examples of the monohydric alcohol include a monohydric alcohol having 1 to 30 carbon atoms, and examples thereof include methanol, ethanol, 1-propanol, 2-propanol, 1-butanol, 2-butanol, and 2-methyl-2-propanol (tert-butyl alcohol). Other examples of the monohydric alcohol include lauryl alcohol, myristyl alcohol, cetanol, cetearyl alcohol, stearyl alcohol, arachidyl alcohol, and behenyl alcohol as saturated aliphatic alcohols, oleyl alcohol and the like as an unsaturated aliphatic alcohol, and hexyldecanol, isostearyl alcohol, octyldodecanol, and decyltetradecanol as aliphatic alcohols including a branched chain. Examples of the polyhydric alcohol include a polyhydric alcohol having 1 to 30 carbon atoms. In addition, examples of the polyhydric alcohol include dihydric alcohols (e.g., ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, and octylene glycol); trihydric alcohols (e.g., glycerin and trimethylolpropane); tetrahydric alcohols (e.g., pentaerythritol such as 1,2,6-hexanetriol); pentahydric alcohols (e.g., xylitol); hexahydric alcohols (e.g., sorbitol and mannitol); polyhydric alcohol polymers (e.g., diethylene glycol, dipropylene glycol, triethylene glycol, polypropylene glycol, tetraethylene glycol, diglycerin, polyethylene glycol, triglycerin, tetraglycerin, and polyglycerin); dihydric alcohol alkyl ethers (e.g., ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, and ethylene glycol dibutyl ether); dihydric alcohol alkyl ethers (e.g., diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether); dihydric alcohol ether esters (e.g., ethylene glycol monomethyl ether acetate, ethylene glycol monoethyl ether acetate, ethylene glycol monobutyl ether acetate, ethylene glycol monophenyl ether acetate, ethylene glycol diadibate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate); glycerin monoalkyl ethers (e.g., xyl alcohol, selachyl alcohol, and batyl alcohol); sugar alcohols (e.g., sorbitol, maltitol, maltotriose, mannitol, sucrose, erythritol, glucose, fructose, starch decomposition sugar, maltose, xylitose, and starch decomposition sugar reducing alcohol); glysolid; tetrahydrofurfuryl alcohol; POE-tetrahydrofurfuryl alcohol; POP-butyl ether; POP/POE-butyl ether; tripolyoxypropylene glycerin ether; POP-glycerin ether; POP-glycerin ether phosphoric acid; POP/POE-pentaerythritol ether; and polyglycerin, and among these, glycerin is particularly preferable. Examples of the sugar alcohol include each of the sugar alcohols described above.

The pH of the active oxygen scavenger of the present disclosure is not particularly limited, and may be, for example, 1.00 or more, 1.50 or more, 2.00 or more, 2.50 or more, 3.00 or more, 3.50 or more, 4.00 or more, 4.50 or more, 5.00 or more, or 5.50 or more; may be, for example, 6.00 or less, 5.50 or less, 5.00 or less, 4.50 or less, 4.00 or less, 3.50 or less, 3.00 or less, 2.50 or less, 2.00 or less, or 1.50 or less; and may be, for example, 1.00 to 6.00, 1.00 to 5.50, 1.00 to 5.00, 1.00 to 4.50, 1.00 to 4.00, 1.00 to 3.50, 1.00 to 3.00, 1.00 to 2.50, 1.00 to 2.00, 1.00 to 1.50, 1.50 to 6.00, 1.50 to 5.50, 1.50 to 5.00, 1.50 to 4.50, 1.50 to 4.00, 1.50 to 3.50, 1.50 to 3.00, 1.50 to 2.50, 1.50 to 2.00, 2.00 to 6.00, 2.00 to 5.50, 2.00 to 5.00, 2.00 to 4.50, 2.00 to 4.00, 2.00 to 3.50, 2.00 to 3.00, 2.00 to 2.50, 2.50 to 6.00, 2.50 to 5.50, 2.50 to 5.00, 2.50 to 4.50, 2.50 to 4.00, 2.50 to 3.50, 2.50 to 3.00, 3.00 to 6.00, 3.00 to 5.50, 3.00 to 5.00, 3.00 to 4.50, 3.00 to 4.00, 3.00 to 3.50, 3.50 to 6.00, 3.50 to 5.50, 3.50 to 5.00, 3.50 to 4.50, 3.50 to 4.00, 4.00 to 6.00, 4.00 to 5.50, 4.00 to 5.00, 4.00 to 4.50, 4.50 to 6.00, 4.50 to 5.50, 4.50 to 5.00, 5.00 to 6.00, 5.00 to 5.50, or 5.50 to 6.00.

The method for producing an active oxygen scavenger of the present disclosure includes oxygen-treating an alcohol with an oxygen allotrope, as described above. However, the active oxygen scavenger of the present disclosure is not limited to this production method, and may be produced by any production method. The method for producing an active oxygen scavenger of the present disclosure will be described below more specifically with reference to examples.

Hereinafter, a case of ozone-oxidizing using glycerin as the alcohol will be described as an example. However, the following description is an exemplification as described above, and the method for producing the oxidation reaction product is not limited to the following description. For example, the following method can be similarly performed by using another alcohol instead of glycerin. The oxygen-treating may be, for example, treating with an oxygen allotrope-containing gas including an oxygen allotrope (e.g., O₂) other than ozone, as described above. In addition, for example, not only the type of alcohol and the method for oxygen-treating the alcohol, but also the concentration of each substance, the reaction temperature, the reaction time, and other reaction conditions can be appropriately changed.

The active oxygen scavenger of the present disclosure can be produced by a production method including oxidizing glycerin (corresponding to the "oxygen-treating") by, for example, bringing glycerin into contact with ozone, more specifically, by mixing glycerin and ozone. The oxidizing glycerin may be, for example, oxidizing glycerin by bringing a glycerin solution and a gas including ozone into gas-liquid contact.

The concentration of the glycerin solution is not particularly limited. As for the glycerin solution, for example, a relatively higher glycerin concentration allows ozone to be treated at a higher concentration, but in the present disclosure, even a low glycerin concentration is sufficiently effective. The concentration of the glycerin solution may be, for example, 1 wt% or more, 2 wt% or more, 3 wt% or more, 5 wt% or more, 10 wt% or more, 20 wt% or more, 30 wt% or more, 40 wt% or more, or 50 wt% or more, and the upper limit is not particularly limited, and the glycerin solution may be, for example, 100 wt% (purity 100%) glycerin. As the high-concentration glycerin solution, for example, a glycerin solution having a glycerin concentration of 75% or more can be used. As specific examples, a glycerin solution of a Japanese Pharmacopoeia product having a glycerin concentration of 84 to 87 wt% is preferable, a concentrated glycerin solution of a Japanese Pharmacopoeia product having a glycerin concentration of 98 wt% or more is more preferable, and a purified glycerin solution having a concentration of 98.5 wt% or more is further preferable. In the glycerin solution, a solvent for glycerin is not particularly limited, and is, for example, an aqueous solvent such as water.

The ozone concentration of the gas including ozone is not particularly limited, and the gas may be, for example, a gas including ozone at a high concentration. The method for producing the gas including ozone is not particularly limited, and for example, an ozone generator that generates ozone by subjecting an oxygen gas to silent discharge can be used. In a case of using an oxygen gas, for example, an oxygen cylinder for medical use may be used, or an oxygen gas produced by an oxygen generator may be used.

The method for bringing the glycerin solution and the gas including ozone into gas-liquid contact is not particularly limited, and includes, for example, a method of putting a high-concentration (for example, 98 wt% or more) glycerin solution into a tank and discharging the gas having a high ozone concentration as fine bubbles into the tank using a gas diffusion tube. Specifically, for example, by aerating a gas having an ozone concentration of about 37,000 ppm into the concentrated glycerin solution for about 7 days, an ozone-treated glycerin solution of about 4,000 ppm in terms of a hydrogen peroxide concentration can be produced. The aeration time is not particularly limited. For example, a relatively longer aeration time allows an ozone-treated glycerin solution including the ozone-treated glycerin having an active oxygen scavenging function at a higher concentration to be produced.

### [2. Alcohol]

The active oxygen scavenger of the present disclosure may be, for example, an alcohol solution. The alcohol is particularly preferably glycerin, as described above. The active oxygen scavenger of the present disclosure can stably retain the oxygen-treated alcohol having an active oxygen scavenging function by, for example, allowing the oxygen-treated alcohol having the active oxygen scavenging function to coexist with an alcohol.

In the composition of the present disclosure, the content of the alcohol is, for example, 0.1 to 100 w/v%, and preferably 40 to 100 w/v%, in a case where the alcohol is glycerin.

### [3. Other Components]

The active oxygen scavenger of the present disclosure may include other components other than the oxygen-treated alcohol treated with the oxygen allotrope. The other components may be, for example, the alcohol, or may be, for example, other components to be added to a dosage form applied to skin. Specific examples of the other components include components to be added to an external preparation for skin and components to be added to a cosmetic (including a quasi-drug). Examples of the component to be added to the external preparation for skin include a pharmaceutically acceptable carrier. Examples of the other component include a component added when applied to skin or applied transdermally, and specific examples thereof include an aqueous solvent such as water, an alcohol, a humectant, an oil, a softener (emollient), a surfactant (solubilizer and emulsifier), a salt, a thickener, a pH adjuster, an ultraviolet absorber, a drug, a buffer, a coloring agent, a preservative, and a fragrance.

In the active oxygen scavenger of the present disclosure, as the other component, for example, an anionic surfactant, a cationic surfactant, an amphoteric surfactant, a nonionic surfactant, a humectant, a water-soluble polymer, a thickener, a film-forming agent, an ultraviolet absorber, a metal ion sequestering agent, a lower alcohol, a polyhydric alcohol, an oil, a sugar, an amino acid, an organic amine, a polymer emulsion, a pH adjuster, a skin nutrient, a vitamin, a plant extract, an antioxidant, an antioxidant aid, a fragrance, a coloring agent, a preservative, a bactericide, water, and the like may be appropriately blended as needed.

Examples of the cationic surfactant include an alkyltrimethylammonium salt (e.g., stearyltrimethylammonium chloride and behenyltrimethylammonium chloride); an alkylpyridinium salt (e.g., cetylpyridinium chloride); distearyldimethylammonium chloride and a dialkyldimethylammonium salt; poly(N,N'-dimethyl-3,5-methylenepiperidinium) chloride; an alkyl quaternary ammonium salt; an alkyldimethylbenzylammonium salt; an alkylisoquinolinium salt; a dialkylmorpholinium salt; a POE-alkylamine; an alkylamine salt; a polyamine fatty acid derivative; an amyl alcohol fatty acid derivative; benzalkonium chloride; and benzethonium chloride.

Examples of the amphoteric surfactant include an imidazoline-based amphoteric surfactant (e.g., sodium 2-undecyl-N,N,N-(hydroxyethylcarboxymethyl)-2-imidazoline, 2-cocoyl-2-imidazolinium hydroxide-1-carboxyethyloxy disodium salt, and 2-heptadecyl-N-carboxymethyl-N-hydroxyethylimidazolinium betaine); and a betaine-based surfactant (e.g., lauryldimethylaminoacetic acid betaine, an alkylbetaine, an alkylamidobetaine, and an alkylsulfobetaine).

Examples of the lipophilic nonionic surfactant include sorbitan fatty acid esters (e.g., sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexanoate, and diglycerol sorbitan tetra-2-ethylhexanoate); glycerin polyglycerin fatty acids (e.g., cottonseed oil fatty acid monoglycerin, glycerin monoerucate, glycerin sesquioleate, glycerin monostearate, glycerin α,α'-oleate pyroglutamate, and glycerin monostearate malate); propylene glycol fatty acid esters (e.g., propylene glycol monostearate); a hydrogenated castor oil derivative; and a glycerin alkyl ether.

Examples of the hydrophilic nonionic surfactant include POE-sorbitan fatty acid esters (e.g., POE-sorbitan monooleate, POE-sorbitan monostearate, POE-sorbitan monooleate, and POE-sorbitan tetraoleate); POE-sorbitol fatty acid esters (e.g., POE-sorbitol monolaurate, POE-sorbitol monooleate, POE-sorbitol pentaoleate, and POE-sorbitol monostearate); POE-glycerin fatty acid esters (e.g., POE-monooleates such as POE-glycerin monostearate, POE-glycerin monoisostearate, and POE-glycerin triisostearate); POE-fatty acid esters (e.g., POE-distearate, POE-monodioleate, and ethylene glycol distearate); POE-alkyl ethers (e.g., POE-lauryl ether, POE-oleyl ether, POE-stearyl ether, POE-behenyl ether, POE-2-octyldodecyl ether, and POE-cholestanol ether); PLURONICs (PLURONIC^{®} (registered trademark)); POE/POP-alkyl ethers (e.g., POE/POP-cetyl ether, POE/POP-2-decyltetradecyl ether, POE/POP-monobutyl ether, POE/POP-hydrogenated lanolin, and POE/POP-glycerin ether); tetra-POE/tetra-POP-ethylenediamine condensates (e.g., TETRONIC); POE-castor oil/hydrogenated castor oil derivatives (e.g., POE-castor oil, POE-hydrogenated castor oil, POE-hydrogenated castor oil monoisostearate, POE-hydrogenated castor oil triisostearate, POE-hydrogenated castor oil monopyroglutamate monoisostearate diester, and POE-hydrogenated castor oil maleic acid); POE-beeswax/lanolin derivatives (e.g., POE-sorbitol beeswax); alkanolamides (e.g., coconut oil fatty acid diethanolamide, lauric acid monoethanolamide, fatty acid isopropanolamide, and cocamide methyl monoethanolamide); POE-propylene glycol fatty acid ester; POE-alkylamine; POE-fatty acid amide; diethylene glycol laurate; sucrose fatty acid ester; alkylethoxydimethylamine oxide; and trioleyl phosphate.

Examples of the humectant include glycerin, a sugar alcohol, polyethylene glycol, propylene glycol, dipropylene glycol, 1,3-butylene glycol, chondroitin sulfate, hyaluronic acid, mucoitin sulfate, caronic acid, atelocollagen, cholesteryl-12-hydroxystearate, sodium lactate, a bile salt, a dl-pyrrolidone carboxylate, an alkylene oxide derivative, a short-chain soluble collagen, a diglycerin (EO) PO adduct, a Rosa roxburghii extract, a yarrow extract, and a melilot extract.

Examples of the natural water-soluble polymer include plant-based polymers (e.g., gum arabic, tragacanth gum, galactan, guar gum, carob gum, karaya gum, carrageenan, pectin, agar, quince seed (marmelo), algae colloid (brown algae extract), starch (rice, corn, potato, and wheat), and glycyrrhizic acid); microorganism-based polymers (e.g., xanthan gum, dextran, succinoglycan, and pullulan); and animal-based polymers (e.g., collagen, casein, albumin, and gelatin).

Examples of the semisynthetic water-soluble polymer include starch-based polymers (e.g., carboxymethyl starch and methylhydroxypropyl starch); cellulose-based polymers (e.g.,methylcellulose, ethylcellulose, methylhydroxypropylcellulose, hydroxyethylcellulose, sodium cellulose sulfate, hydroxypropylcellulose, carboxymethylcellulose, sodium carboxymethylcellulose, crystalline cellulose, and cellulose powder); and alginic acid-based polymers (e.g., sodium alginate and propylene glycol alginate).

Examples of the synthetic water-soluble polymer include vinyl-based polymers (e.g., polyvinyl alcohol, polyvinyl methyl ether, polyvinylpyrrolidone, and carboxyvinyl polymer); polyoxyethylene-based polymers (e.g., polyoxyethylene polyoxypropylene copolymers of polyethylene glycol 20,000, 40,000, and 60,0000, and a highly polymerized polyethylene glycol); acrylic polymers (e.g., sodium polyacrylate, polyethyl acrylate, and polyacrylamide); polyethyleneimine; and a cationic polymer.

Examples of the thickener include gum arabic, carrageenan, karaya gum, tragacanth gum, carob gum, quince seed (marmelo), casein, dextrin, gelatin, sodium pectate, sodium alginate, methylcellulose, ethylcellulose, CMC, hydroxyethylcellulose, hydroxypropylcellulose, PVA, PVM, PVP, sodium polyacrylate, carboxyvinyl polymer, locust bean gum, guar gum, tamarind gum, dialkyldimethylammonium cellulose sulfate, xanthan gum, aluminum magnesium silicate, bentonite, hectorite, AlMg silicate (VEEGUM), LAPONITE, and silicic anhydride.

Examples of the ultraviolet absorber include benzoic acid-based ultraviolet absorbers (e.g., para-aminobenzoic acid (hereinafter, abbreviated as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, and N,N-dimethyl PABA ethyl ester); anthranilic acid-based ultraviolet absorbers (e.g., homomenthyl-N-acetyl anthranilate); salicylic acid-based ultraviolet absorbers (e.g., amyl salicylate, menthyl salicylate, homomenthyl salicylate, octyl salicylate, phenyl salicylate, benzyl salicylate, and p-isopropanolphenyl salicylate); cinnamic acid-based ultraviolet absorbers (e.g., octyl methoxycinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, octyl-p-methoxycinnamate (2-ethylhexyl-p-methoxycinnamate), 2-ethoxyethyl-p-methoxycinnamate, cyclohexyl-p-methoxycinnamate, ethyl-α-cyano-β-phenylcinnamate, 2-ethylhexyl-α-cyano-β-phenylcinnamate, and glyceryl mono-2-ethylhexanoyl-di-p-methoxycinnamate); benzophenone-based ultraviolet absorbers (e.g., 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, 2-hydroxy-4-n-octoxybenzophenone, and 4-hydroxy-3-carboxybenzophenone); 3-(4'-methylbenzylidene)-d,l-camphor; 3-benzylidene-d,l-camphor; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenyl benzotriazole; dibenzalazine; dianisoylmethane; 4-methoxy-4'-t-butyldibenzoylmethane; 5-(3,3-dimethyl-2-norbornylidene)-3-pentan-2-one; dimorpholinopyridazino; 2-ethylhexyl-2-cyano-3,3-diphenylacrylate; and 2,4-bis-{[4-(2-ethylhexyloxy)-2-hydroxy]-phenyl}-6-(4-methoxyphenyl)-(1,3,5)-triazine.

Examples of the metal ion sequestering agent include 1-hydroxyethane-1,1-diphosphonic acid, 1-hydroxyethane-1,1-diphosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, and trisodium ethylenediamine hydroxyethyl triacetate.

Examples of the lower alcohol include ethanol, propanol, isopropanol, isobutyl alcohol, and t-butyl alcohol.

Examples of the polyhydric alcohol include each of the polyhydric alcohols described above.

Examples of the monosaccharide include trioses (e.g., D-glyceraldehyde and dihydroxyacetone); tetroses (e.g., D-erythrose, D-erythrulose, D-threose, and erythritol); pentoses (e.g., L-arabinose, D-xylose, L-lyxose, D-arabinose, D-ribose, D-ribulose, D-xylulose, and L-xylulose); hexoses (e.g., D-glucose, D-talose, D-psicose, D-galactose, D-fructose, L-galactose, L-mannose, and D-tagatose); heptoses (e.g., aldoheptose and heptulose); octoses (e.g., octulose); deoxy sugars (e.g., 2-deoxy-D-ribose, 6-deoxy-L-galactose, and 6-deoxy-L-mannose); amino sugars (e.g., D-glucosamine, D-galactosamine, sialic acid, aminouronic acid, and muramic acid); and uronic acids (e.g., D-glucuronic acid, D-mannuronic acid, L-guluronic acid, D-galacturonic acid, and L-iduronic acid).

Examples of the oligosaccharide include sucrose, gentianose, umbelliferose, lactose, planteose, isolychnoses, α,α-trehalose, raffinose, lychnoses, umbilicin, stachyose, and verbascoses.

Examples of the polysaccharide include cellulose, quince seed, chondroitin sulfate, starch, galactan, dermatan sulfate, glycogen, gum arabic, heparan sulfate, hyaluronic acid, tragacanth gum, keratan sulfate, chondroitin, mucoitin sulfate, guar gum, dextran, keratosulfate, locust bean gum, succinoglycan, and caronic acid.

Examples of the amino acid include neutral amino acids (e.g., threonine and cysteine); and basic amino acids (e.g., hydroxylysine). In addition, examples of the amino acid derivative include sodium acylsarcosine (sodium lauroylsarcosine), an acylglutamate, sodium acyl-β-alanine, glutathione, and pyrrolidone carboxylic acid.

Examples of the organic amine include monoethanolamine, diethanolamine, triethanolamine, morpholine, triisopropanolamine, 2-amino-2-methyl-1,3-propanediol, and 2-amino-2-methyl-1-propanol. Examples of the polymer emulsion include an acrylic resin emulsion, a polyethyl acrylate emulsion, an acrylic resin liquid, a polyalkyl acrylate emulsion, a polyvinyl acetate resin emulsion, and a natural rubber latex.

Examples of the pH adjuster include a buffer such as citric acid, lactic acid-sodium lactate, citric acid-sodium citrate, and succinic acid-sodium succinate. Examples of the vitamins include vitamins A, B1, B2, B6, C, and E, and derivatives thereof, pantothenic acid and a derivative thereof, and biotin. Examples of the antioxidant include tocopherols, dibutylhydroxytoluene, butylhydroxyanisole, and gallic acid esters.

Examples of the antioxidant aid include phosphoric acid, citric acid, ascorbic acid, maleic acid, malonic acid, succinic acid, fumaric acid, cephalin, hexametaphosphate, phytic acid, and ethylenediaminetetraacetic acid.

Examples of other blendable components include preservatives (e.g., ethylparaben, butylparaben, chlorphenesin, and phenoxyethanol); bactericides (e.g., isopropylmethylphenol, benzalkonium chloride, benzethonium chloride, and chlorhexidine gluconate); antiphlogistics (e.g., a glycyrrhizic acid derivative, a glycyrrhetinic acid derivative, a salicylic acid derivative, hinokitiol, zinc oxide, and allantoin); whitening agents (e.g., a placenta extract, a Saxifraga stolonifera extract, and arbutin); various extracts (e.g., Phellodendron bark, Coptis rhizome, Lithospermum root, peony root, Swertia herb, birch, sage, loquat, carrot, aloe, mallow, iris, grape, coix seed, sponge gourd, lily, saffron, Cnidium rhizome, ginger, Hypericum, Ononis, garlic, capsicum, citrus unshiu peel, Angelica acutiloba, and seaweed); activators (e.g., royal jelly, a photosensitizer, and a cholesterol derivative); blood circulation promoters (e.g., nonylic acid vanillylamide, benzyl nicotinate, β-butoxyethyl nicotinate, capsaicin, zingerone, cantharides tincture, ichthammol, tannic acid, α-borneol, tocopherol nicotinate, inositol hexanicotinate, cyclandelate, cinnarizine, tolazoline, acetylcholine, verapamil, cepharanthine, and γ-oryzanol); antiseborrheic agents (e.g., sulfur and thianthol); and anti-inflammatory agents (e.g., tranexamic acid, thiotaurine, and hypotaurine).

Furthermore, metal sequestering agents such as disodium edetate, trisodium edetate, sodium citrate, sodium polyphosphate, sodium metaphosphate, gluconic acid, and malic acid; caffeine; tannin; verapamil; tranexamic acid or a derivative thereof; various herbal extracts such as licorice, *Pseudocydonia sinensis*, and *Pyrola japonica*; drugs such as tocopherol acetate, glycyrrhetinic acid, glycyrrhizic acid or a derivative thereof, or a salt thereof; whitening agents such as vitamin C, magnesium ascorbyl phosphate, ascorbyl glucoside, arbutin, and kojic acid; amino acids such as arginine and lysine, or derivatives thereof; and saccharides such as fructose, mannose, erythritol, trehalose, and xylitol can also be appropriately blended.

### [4. Dosage Form, Usage, and Application]

The active oxygen scavenger of the present disclosure may be, for example, in a solid state or a liquid state. Examples of the form of the composition of the present disclosure include dosage forms such as oral agents and instillations; dosage forms such as tablets and capsules; lotion dosage forms; emulsion dosage forms such as milky lotions and creams; oil dosage forms; gel dosage forms; and ointments, packs, and cleansers.

A subject of use of the active oxygen scavenger of the present disclosure may be, for example, a human or a non-human animal. The active oxygen scavenger of the present disclosure can be used, for example, on the skin of the subject. The use amount of the composition of the present disclosure is not particularly limited, and can be an amount usually used depending on the dosage form of the active oxygen scavenger of the present disclosure.

The active oxygen scavenger of the present disclosure can be suitably applied, for example, to an application of an external preparation for skin such as a cosmetic product. In addition, the application of the active oxygen scavenger of the present disclosure is not limited thereto, and the active oxygen scavenger can be used, for example, in fields such as pharmaceuticals, cosmetic preparations (cosmetic products), foods and drinks, and oral compositions.

### (Pharmaceutical)

The administration form of the pharmaceutical of the present disclosure is not particularly limited. In a case where the composition of the present disclosure is administered in vivo, oral administration or parenteral administration may be used. Examples of the parenteral administration include intravenous injection (intravenous administration), intramuscular injection (intramuscular administration), transdermal administration, subcutaneous administration, intradermal administration, enteral administration, rectal administration, vaginal administration, nasal administration, pulmonary administration, intraperitoneal administration, and local administration.

The dosage form of the pharmaceutical of the present disclosure is not particularly limited, and can be appropriately determined depending on the administration form, for example. Examples of the dosage form include a liquid state and a solid state. As specific examples, examples of the dosage form include preparations for oral administration such as modified-release preparations (e.g., an enteric-coated preparation and a sustained-release preparation), capsules, oral liquid preparations (e.g., an elixir, a suspension, an emulsion, an aromatic water, and a lemonade), syrups (e.g., a preparation for a syrup), granules (e.g., an effervescent granule and a fine granule), powders, tablets (e.g., an orally disintegrating tablet, a chewable tablet, an effervescent tablet, a dispersible tablet, a dissolvable preparation, and a coated tablet), pills, and oral jellies; preparations for oral cavity application such as tablets for oral cavity (e.g., a gum, a sublingual tablet, a troche, a drop, a buccal tablet, and an adhesive tablet), sprays for oral cavity, semi-solid preparations for oral cavity, and gargles; preparations for injection administration such as injections (e.g., an implantable injection, a long-acting injection, an infusion (e.g., a preparation for intravenous drip), a freeze-dried injection, a powder injection, a pre-filled syringe, and a cartridge); preparations for dialysis such as dialysis agents (e.g., a peritoneal dialysis agent and a hemodialysis agent); preparations for bronchial/pulmonary application such as inhalants (e.g., an inhalation aerosol, an inhalation liquid, and an inhalation powder); preparations for ophthalmic administration such as ophthalmic ointments and eye drops; preparations for otic administration such as ear drops; preparations for nasal application such as nasal drops (e.g., a nasal liquid and a nasal powder); preparations for rectal application such as suppositories, semi-solid preparations for rectum, and enemas; preparations for vaginal application such as vaginal suppositories and vaginal tablets; and preparations for skin application such as liquids for external use (e.g., a spirit, a liniment, and a lotion), creams, gels, solids for external use (e.g., a powder for external use), sprays (e.g., an aerosol for external use and a pump spray), patches (e.g., a tape and a cataplasm), and ointments. In a case where the composition of the present disclosure is orally administered, examples of the dosage form include tablets, coated tablets, pills, fine granules, granules, powders, capsules, liquids, syrups, emulsions, and suspensions. In a case where the composition of the present disclosure is parenterally administered, examples of the dosage form include preparations for injection administration and preparations for intravenous drip. In a case where the composition of the present disclosure is transdermally administered, examples of the dosage form include external medicines such as patches, application preparations, ointments, creams, and lotions.

The pharmaceutical of the present disclosure may include, for example, an additive as needed, and in a case where the composition of the present disclosure is used as a medicine or a pharmaceutical composition, the additive preferably includes a pharmaceutically acceptable additive or a pharmaceutically acceptable carrier. The additive is not particularly limited, and examples thereof include a base material, an excipient, a coloring agent, a lubricant, a binder, a disintegrant, a stabilizer, a preservative agent, a pH adjuster, and a flavoring and deodorizing agent such as a fragrance.

Examples of the excipient include sugar derivatives such as lactose, white sugar, glucose, mannitol, and sorbitol; starch derivatives such as corn starch, potato starch, pregelatinized starch, and dextrin; cellulose derivatives such as crystalline cellulose; organic excipients such as acacia gum, dextran, and pullulan; silicate derivatives such as light anhydrous silicic acid, synthetic aluminum silicate, calcium silicate, and magnesium aluminometasilicate; phosphates such as calcium hydrogen phosphate; carbonates such as calcium carbonate; and inorganic excipients such as a sulfate such as calcium sulfate. Examples of the lubricant include metal stearates such as stearic acid, calcium stearate, and magnesium stearate; talc; polyethylene glycol; and a hydrogenated vegetable oil. Examples of the flavoring and deodorizing agent include fragrances such as cocoa powder, menthol, aromatic powder, peppermint oil, borneol, and cinnamon powder, a sweetener, and an acidulant. Examples of the binder include hydroxypropylcellulose, hydroxypropylmethylcellulose, polyvinylpyrrolidone, and macrogol. Examples of the disintegrant include cellulose derivatives such as carboxymethylcellulose and calcium carboxymethylcellulose; and chemically modified starches and chemically modified celluloses such as carboxymethyl starch, sodium carboxymethyl starch, and cross-linked polyvinylpyrrolidone. Examples of the stabilizer include p-hydroxybenzoates such as methylparaben and propylparaben; alcohols such as chlorobutanol, benzyl alcohol, and phenylethyl alcohol; benzalkonium chloride; phenols such as phenol and cresol; thimerosal; dehydroacetic acid; and sorbic acid. The pH adjuster can be appropriately determined depending on a target pH, for example. As a specific example, in a case of adjusting to an acidic side, an acidic substance such as acetic acid, lactic acid, phosphoric acid, tartaric acid, citric acid, ascorbic acid, hydrochloric acid, gluconic acid, and sulfuric acid can be used as the pH adjuster, for example. In a case of adjusting to a basic side, a basic substance such as potassium hydroxide, sodium hydroxide, calcium hydroxide, magnesium hydroxide, monoethanolamine, diethanolamine, and triethanolamine can be used as the pH adjuster, for example. The pH adjuster may be, for example, used singly, or two or more types thereof may be used in combination.

### (Cosmetic Preparations)

The cosmetic preparations of the present disclosure are suitably used, for example, as skin (skin care) cosmetic preparations and/or hair (hair care) cosmetic preparations by having excellent safety, stability, and usability. Examples of the skin care cosmetic preparations include skin cosmetic preparations such as a skin lotion, a milky lotion, a cream, a serum, and a pack; base cosmetic preparations such as a cleansing product, a facial wash, an ultraviolet inhibitor, a makeup, a base lotion, and a makeup base cream; makeup cosmetic preparations such as foundations in various dosage forms such as a milky lotion preparation, an oily preparation, and a solid form, an eye color, and a cheek color; and body cosmetic preparations such as a hand cream, a leg cream, a neck cream, and a body lotion.

Examples of the hair care cosmetic preparations include shampoos such as an oil shampoo, a cream shampoo, a conditioning shampoo, a shampoo for dandruff, a shampoo for hair color, and a rinse-integrated shampoo; a rinse, a treatment, a hair pack, a hair foam, a hair mousse, a hair spray, a hair mist, a hair wax, a hair gel, a water grease, a set lotion, a color lotion, a hair tonic, a hair liquid, a pomade, a stick pomade, a hair cream, a hair blow, a split end coat, a hair oil, a preparation for permanent wave, a straight perm preparation, an oxidation hair dye, a hair bleach, a hair color pre-treatment, a hair color after-treatment, a perm pre-treatment, a perm after-treatment, a hair manicure, and a hair grower.

The cosmetic preparations of the present disclosure are suitably applied to humans, and can also be applied to animals other than humans as long as the respective operational effects are exhibited.

### (Foods and Drinks)

The foods and drinks of the present disclosure contain, for example, the active oxygen scavenger of the present disclosure, and further contain other components as needed. The ozone-treated glycerin included in the active oxygen scavenger of the present disclosure has, for example, high safety performance, and has almost no effect on other components. Here, the foods and drinks refer to those that have little risk of harming human health and are ingested by oral or gastrointestinal administration in normal social life, and are not limited to categories such as foods, pharmaceuticals, and quasi-drugs in terms of administrative categories. Therefore, the "foods and drinks" in the present embodiment broadly include a general food, a health food (functional food and drink), a food with health claims (food for specified health uses, food with nutrient function claims), a quasi-drug, a pharmaceutical, and the like that are orally ingested. The content of the ozone-treated glycerin in the foods and drinks is not particularly limited, and can be appropriately adjusted.

### (Other Components)

The other components included in the foods and drinks of the present disclosure are not particularly limited, and can be appropriately selected from auxiliary raw materials, additives, or other components used in the production of normal foods and drinks depending on the purpose. Examples thereof include glucose, fruit sugar, sucrose, maltose, sorbitol, stevioside, rubusoside, corn syrup, lactose, an oligosaccharide, xylitol, trehalose, palatinose, aspartame, acesulfame potassium, sucralose, saccharin salts, citric acid, tartaric acid, malic acid, succinic acid, lactic acid, L-ascorbic acid, dl-α-tocopherol, sodium erythorbate, glycerin, propylene glycol, a glycerin fatty acid ester, a polyglycerin fatty acid ester, a sucrose fatty acid ester, a sorbitan fatty acid ester, gum arabic, carrageenan, casein, gelatin, pectin, agar, B vitamins, nicotinamide, calcium pantothenate, amino acids, calcium salts, a pigment, a fragrance, and a preservative agent. These may be used singly, or two or more types thereof may be used in combination. The blending amount of the other components is not particularly limited, and can be appropriately selected depending on the purpose.

The foods and drinks are not particularly limited, and specific examples thereof include beverages such as a refreshing beverage, a carbonated drink, a nutritional drink, a fruit drink, and a lactic acid drink (including concentrated stock solutions and adjusting powders of these beverages); frozen desserts such as an ice cream, an ice sherbet, and a shaved ice; noodles such as a soba noodle, an udon noodle, a glass noodle, a dumpling skin, a shumai skin, a Chinese noodle, and an instant noodle; confectioneries such as a sweet, a chewing gum, a candy, a gum, a chocolate, a tablet candy, a snack confectionery, a biscuit, a jelly, a jam, a cream, and a baked confectionery; processed marine and livestock foods such as a kamaboko, a ham, and a sausage; dairy products such as a processed milk and a fermented milk; fats and oils and processed fat and oil foods such as a salad oil, a tempura oil, a margarine, a mayonnaise, a shortening, a whipped cream, and a dressing; seasonings such as a sauce and a dipping sauce; a soup, a stew, a salad, a side dish, and a pickle; health and nutritional supplements in other various forms; tablets; capsules, and drinkable preparation.

As an embodiment of the foods and drinks, a well-known food or pharmaceutical-like form can be adopted. For example, as the pharmaceutical-like form, forms of oral medicines such as a powder, a capsule, a granule, a tablet, a liquid, and others can also be adopted. In addition, as what is in the form of a normal food, it can be made into a jelly, a syrup, a sweet, a gum, a soft drink, a supplement, and other well-known food forms, or a predetermined amount can be mixed into other well-known foods.

The intake method of the foods and drinks is not particularly limited, and can be appropriately selected depending on the purpose. Examples thereof include oral administration, parenteral administration, and gastrointestinal administration. Among these, oral administration is preferable.

The foods and drinks of the present disclosure are suitably applied to humans, but can also be applied to animals other than humans as long as the respective operational effects are exhibited.

### (Oral Compositions)

The oral compositions of the present disclosure contain, for example, the active oxygen scavenger of the present disclosure, and further contain other components as needed. The ozone-treated glycerin included in the active oxygen scavenger of the present disclosure has, for example, high safety performance, and has almost no effect on other components. The content of the ozone-treated glycerin in the oral compositions is not particularly limited, and can be appropriately adjusted.

The application form of the oral compositions is not particularly limited, and the oral compositions can be used, for example, as pharmaceuticals, quasi-drugs, and cosmetic products. As the application of the oral compositions, known ones can be appropriately adopted. Examples of the application include a chewing preparation, a dissolvable preparation for oral cavity, a preparation for oral cavity disintegration, a tongue care preparation, a mouth refresher, a toothpaste, a mouthwash, a gargle, a liquid toothpaste, a biofilm dispersant, a bad breath preventive, a gum massage preparation, a wetting agent for oral cavity, a tongue coating remover, a preparation for oral cavity application, an oral cavity bactericide, a throat bactericide, an oral cavity and throat preparation, a therapeutic agent for periodontal disease, a denture wearing agent, a denture coating agent, a denture stabilizer, a denture preservative, a denture cleanser, and an implant care agent.

The dosage form of the oral compositions is not particularly limited, and by containing a solvent such as water and an alcohol, the oral compositions can be applied to, for example, an ointment, a paste, a pasta, a spray, a gel, a liquid, a suspension, and a gum.

The oral compositions may contain other components other than the components described above depending on the application purpose, form, application, and the like. Examples of the other components include an antibacterial agent, an anti-inflammatory agent, a fragrance, a wetting agent, an abrasive, alcohols, a thickener, a sweetening component, a medicinal component, a coloring agent, a stabilizer, and a pH adjuster. As the other components, known ones blended in oral compositions can be used. The oral compositions may contain only one type of each of the other components described above singly, or may contain two or more types thereof in combination.

### Examples

Next, Examples of the present disclosure will be described. However, the present disclosure is not limited by the following Examples. Commercially available reagents were used based on their protocols unless otherwise specified. In addition, in the following Examples, in a case where the concentration of a solution is represented by "%", it is wt% (w/v) unless otherwise specified.

### [Example 1]

A glycerin solution including ozone-treated glycerin was produced by ozone oxidation of glycerin. Furthermore, the active oxygen scavenging function of the produced ozone-treated glycerin solution was verified. The present Example corresponds to an Example in which the active oxygen scavenger of the present disclosure is produced and used, corresponds to an Example in which the active oxygen scavenging method of the present disclosure is used, and further corresponds to an Example of the method for producing an active oxygen scavenger of the present disclosure.

### (1) Production of Ozone-Treated Glycerin Solution Including Ozone-Treated Glycerin

Concentrated glycerin and ozone were brought into gas-liquid contact, and an ozone-treated glycerin solution including ozone-treated glycerin was produced as described below. Here, the concentrated glycerin means glycerin of a Japanese Pharmacopoeia product having a concentration of 98 wt% or more.

As a contact tank, a tank made of Teflon (registered trademark) having a capacity of 50 L was used. A gas diffusion tube was installed on a bottom of the tank so that ozone could be supplied into the tank as fine bubbles. A silent discharge type ozone generator having an ozone generation capacity of 100 g/hour was used, using a high-concentration oxygen gas including high-concentration oxygen of 90% by volume or more as a raw material.

22 kg of the concentrated glycerin was put into the tank, the high-concentration oxygen gas was fed into the ozone generator at 20 L/min to generate a gas including ozone, and the generated gas was discharged from the gas diffusion tube into the tank for a predetermined time, to obtain a glycerin solution in which ozone-treated glycerin was dissolved.

### (2) Verification of Active Oxygen Scavenging Function

An ESR spectrum of an OH radical generated by a Fenton reaction-DMPO spin trap method for H₂O₂ was measured. Furthermore, an ESR measurement was performed when various samples (test substances) were added and mixed, and the active oxygen scavenging action of the samples was evaluated by comparing a spectrum intensity of DMPO-OH. In addition, in the following, "OG" represents ozonated glycerin.

### Experimental conditions:

- ESR apparatus: EMX-nano (Bruker)
- OG or H₂O₂ alone
   DMPO 5 µL
   Purified water 140 µL
   1 mmol/L FeSO₄ 20 µL
   Sample 20 µL
- Mixed sample of OG and H₂O₂ (1:1)
   DMPO 5 µL
   Purified water 120 µL
   1 mmol/L FeSO₄ 20 µL
   Sample 40 µL
- In a case where the sample had no spectrum, the spectra of [Sample + H₂O₂ mix (mixture of the sample and H₂O₂)] and [H₂O₂] were compared.
- In a case where the sample included a spectrum such as DMPO-OH, the spectrum of [Sample + H₂O₂ mix] - [Sample] (spectrum subtraction processing) and the spectrum of [H₂O₂] were compared. From these, the active oxygen scavenging rate was calculated. In a case where the OG concentration is high, the apparent spectrum intensity changes in a portion where the six-line spectrum of OG and the four-line spectrum of DMPO-OH derived from H₂O₂ overlap. Therefore, by subtracting the spectrum of [OG] from the spectrum of [OG + H₂O₂ mix], only the spectrum of H₂O₂ in the [OG + H₂O₂ mix] sample was extracted, and the evaluation was performed using this. In addition, in the case of subtracting the spectrum of [OG] from the spectrum of [OG + H₂O₂ mix], the spectrum intensity of the first line from the left of the six lines of the OG spectrum was corrected to be the same.

In (1) described above, when ozone was discharged into the tank for 5 days, ozone-treated glycerin having an oxidizing power of 2,000 ppm in terms of ozone concentration (hereinafter, sometimes referred to as "OG2000") was obtained. What was obtained by diluting this with water to 1/10, 1/100, and 1/500 are hereinafter sometimes referred to as OG200, OG20, and OG4, respectively.

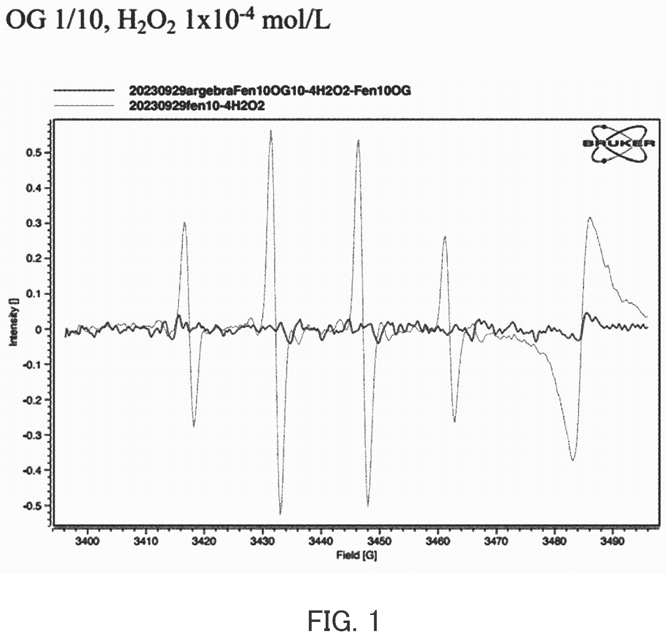

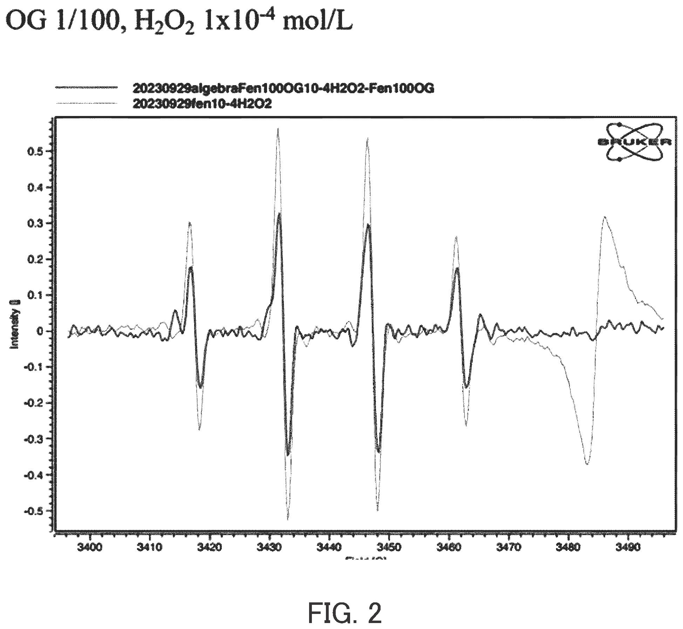

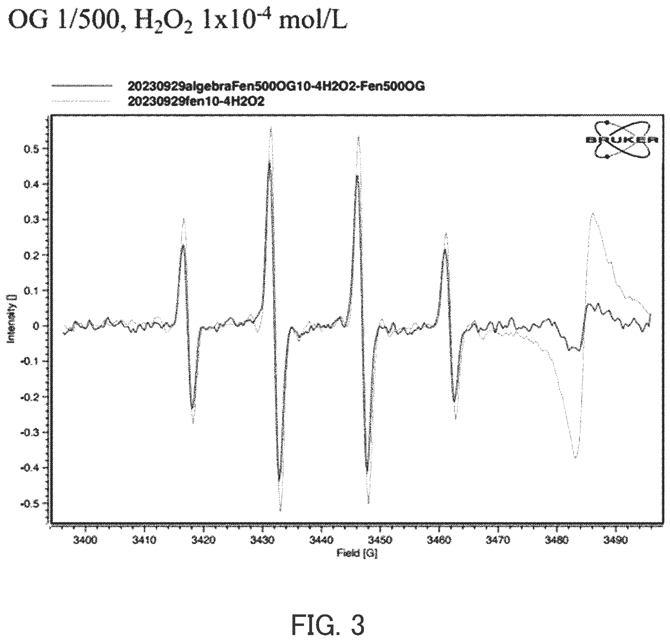

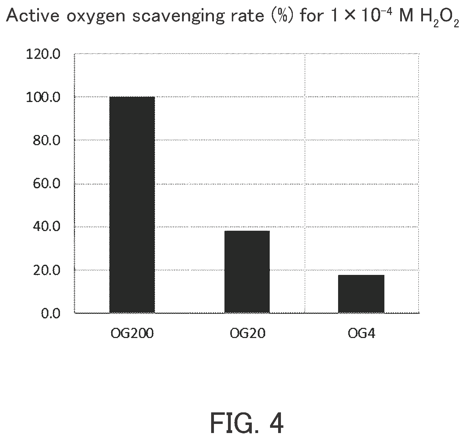

FIGs. 1 to 3 show ESR spectra of OG200, OG20, and OG4 measured by the measurement method of (2) described above. In FIG. 1, "OG 1/10" is synonymous with OG200. In FIG. 2, "OG 1/100" is synonymous with OG20. In FIG. 3, "OG 1/500" is synonymous with OG4. The bar graph of FIG. 4 shows the active oxygen scavenging rates (%) calculated from these ESR spectra. The numerical value of this active oxygen scavenging rate is an index representing the highness of the active oxygen scavenging function. In addition, the numerical values in FIG. 4 are numerical values with the active oxygen scavenging rate of OG200 as 100%. As shown in FIG. 4, the higher the concentration of the ozone-treated glycerin, the higher the active oxygen scavenging rate was shown.

Next, the active oxygen scavenging rates of the ozone-treated glycerin and the untreated glycerin were compared. Specifically, the active oxygen scavenging rate was compared between each of the following commercially available glycerins diluted to 1/10 with water and OG200 obtained by diluting OG2000 to 1/10 with water, by the measurement method of (2) described above.

### Test substances:

Synthetic glycerin (Spectrum)
Concentrated glycerin for cosmetics (Kao Corporation)
Japanese Pharmacopoeia concentrated glycerin (Kao Corporation)
Japanese Pharmacopoeia concentrated glycerin (Sakamoto Yakuhin Kogyo Co., Ltd.)
Reagent special grade glycerin (FUJIFILM Wako Pure Chemical Corporation)
Ozone-treated glycerin 200 ppm

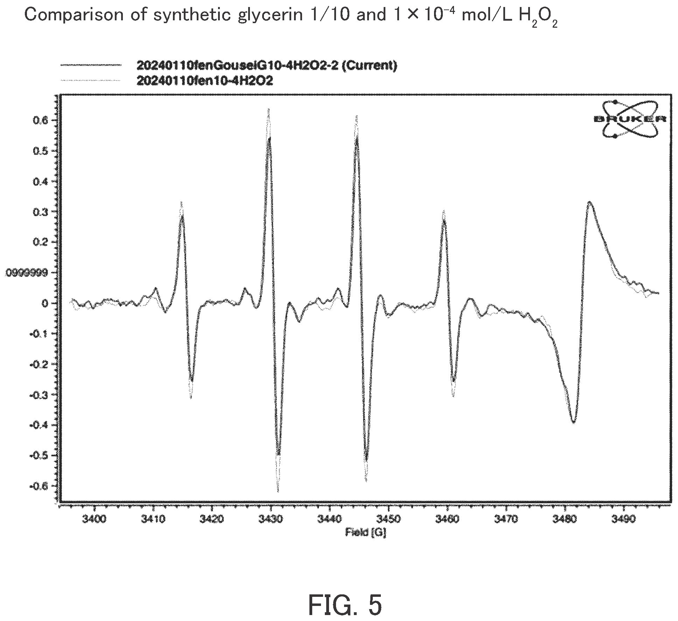

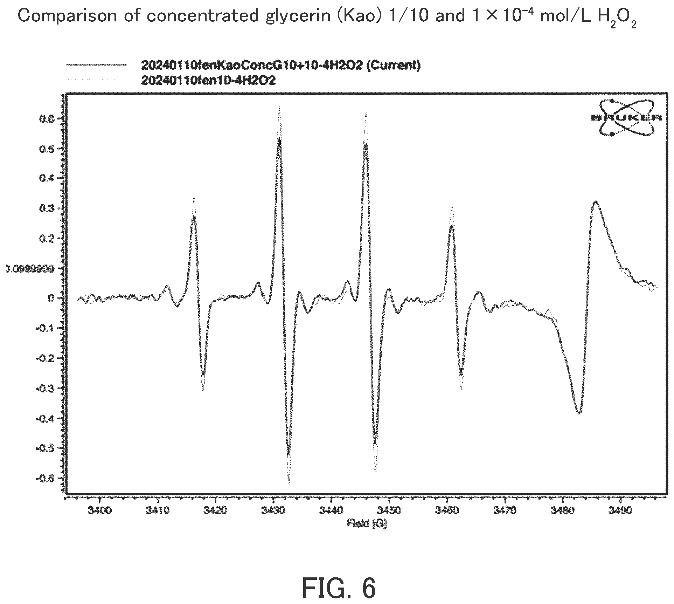

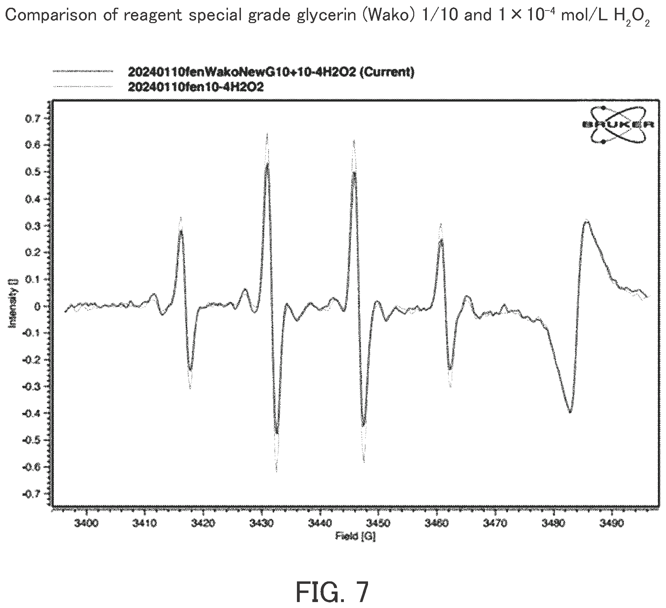

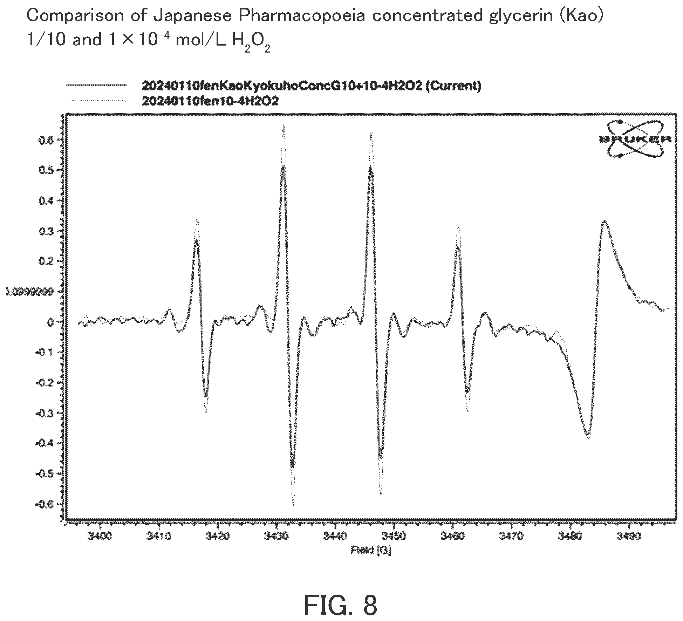

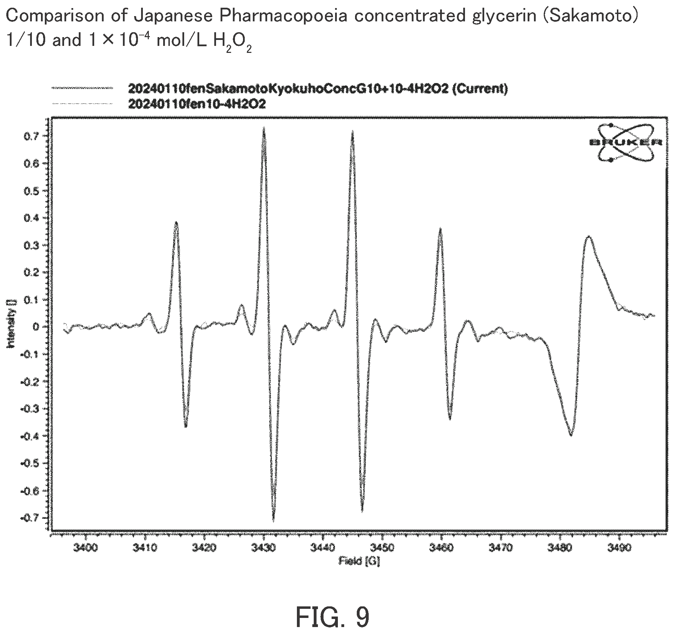

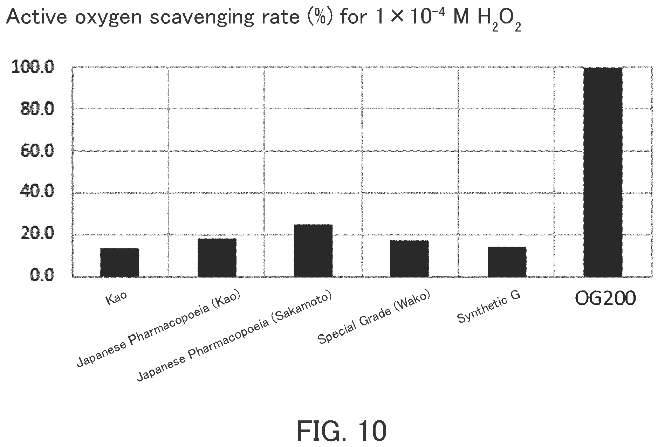

FIGs. 5 to 9 show the ESR spectra of the synthetic glycerin, the concentrated glycerin for cosmetics (Kao Corporation), the reagent special grade glycerin (FUJIFILM Wako Pure Chemical Corporation), the Japanese Pharmacopoeia concentrated glycerin (Kao Corporation), and the Japanese Pharmacopoeia concentrated glycerin (Sakamoto Yakuhin Kogyo Co., Ltd.) through the measurement method of (2) described above, respectively. The ESR spectrum of OG200 is as shown in FIG. 1. In addition, the bar graph of FIG. 10 shows the active oxygen scavenging rates (%) calculated from these ESR spectra. As shown in FIG. 10, the active oxygen scavenging rate of the untreated glycerin was extremely low as compared with the ozone-treated glycerin, and therefore it could be verified that the ozone-treated glycerin has a high active oxygen scavenging function.

### [Example 2]

An oxygen-treated glycerin solution including oxygen-treated glycerin was produced in the same manner as in (1) of Example 1, except that an oxygen cylinder was used instead of the silent discharge type ozone generator of Example 1, and glycerin was ozone-treated using oxygen (O₂) in this oxygen cylinder as a raw material. Furthermore, the active oxygen scavenging function of the produced oxygen-treated glycerin solution was verified in the same manner as in (2) of Example 1. The present Example corresponds to an Example in which the active oxygen scavenger of the present disclosure is produced and used, corresponds to an Example in which the active oxygen scavenging method of the present disclosure is used, and further corresponds to an Example of the method for producing an active oxygen scavenger of the present disclosure.

### [Example 3]

An oxygen-treated glycerin solution including oxygen-treated glycerin was produced in the same manner as in (1) of Example 1, except that air was used instead of the silent discharge type ozone generator of Example 1, and glycerin was ozone-treated using oxygen (O₂) in this air as a raw material. Furthermore, the active oxygen scavenging function of the produced oxygen-treated glycerin solution was verified in the same manner as in (2) of Example 1. The present Example corresponds to an Example in which the active oxygen scavenger of the present disclosure is produced and used, corresponds to an Example in which the active oxygen scavenging method of the present disclosure is used, and further corresponds to an Example of the method for producing an active oxygen scavenger of the present disclosure.

### [Comparison of Active Oxygen Scavenging Rates of Examples 1 To 3]

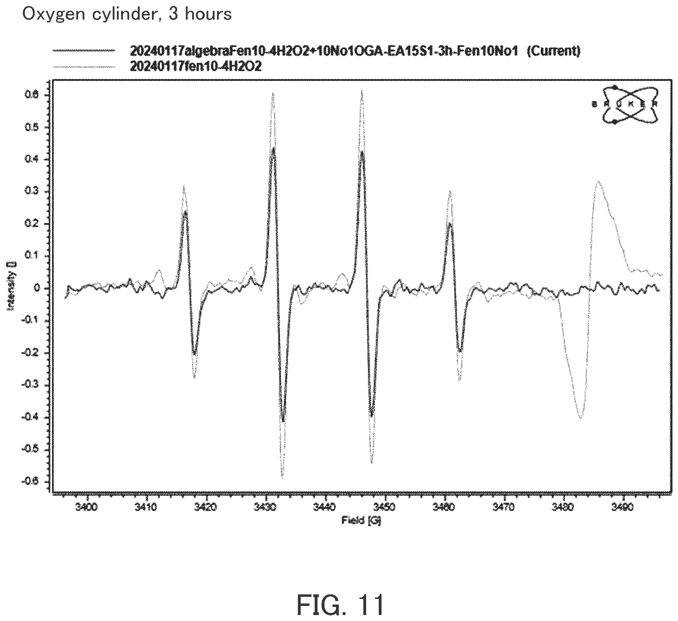

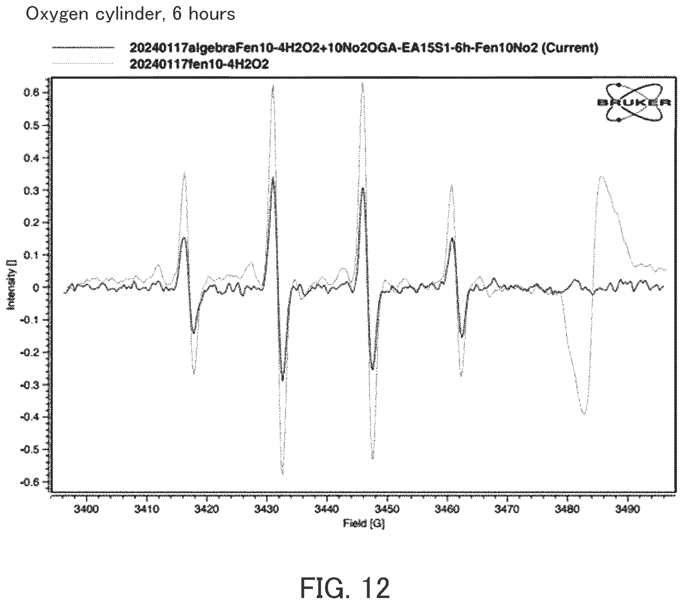

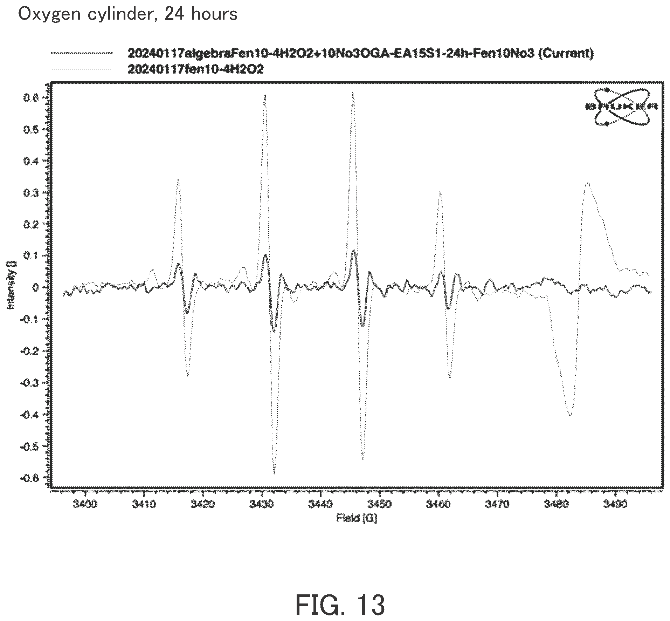

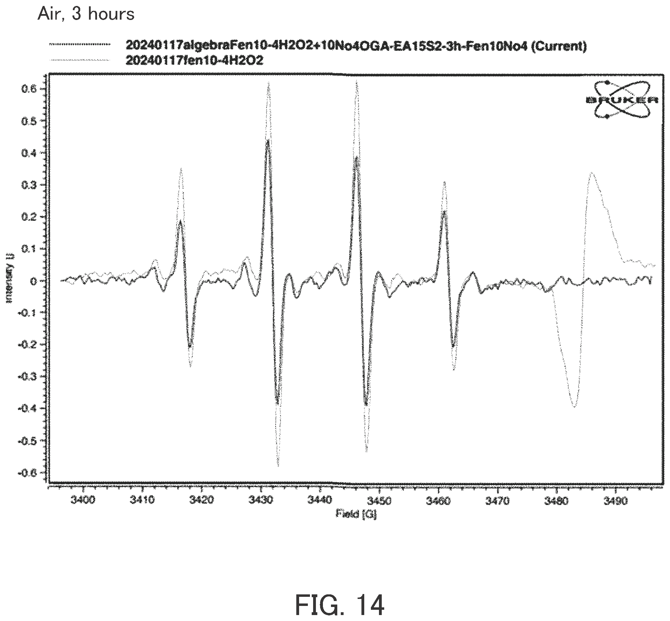

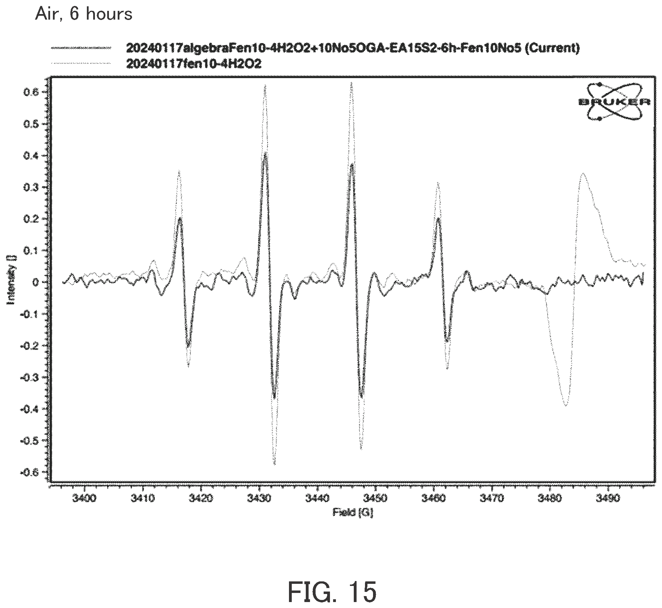

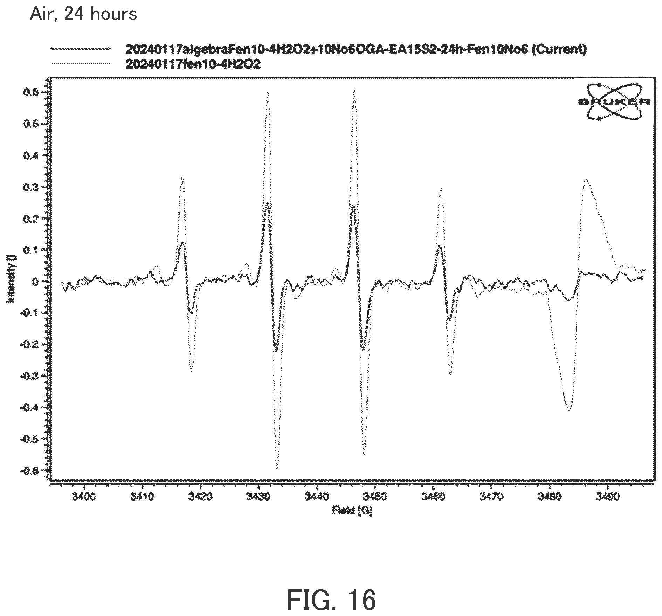

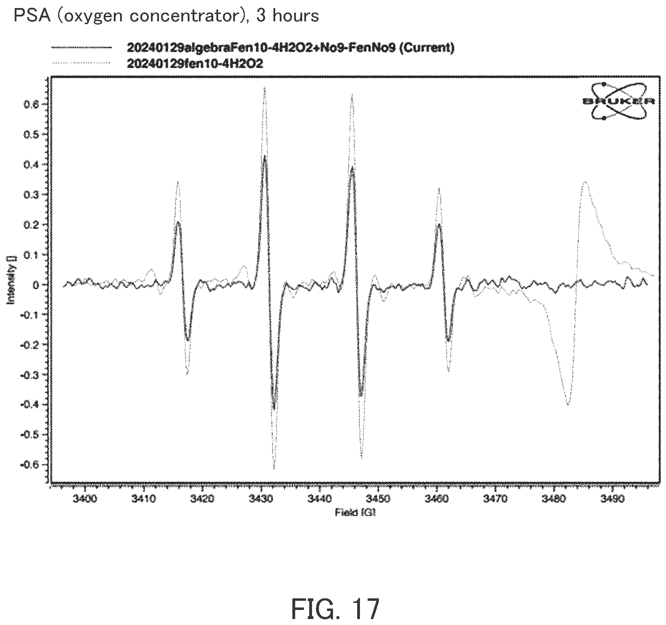

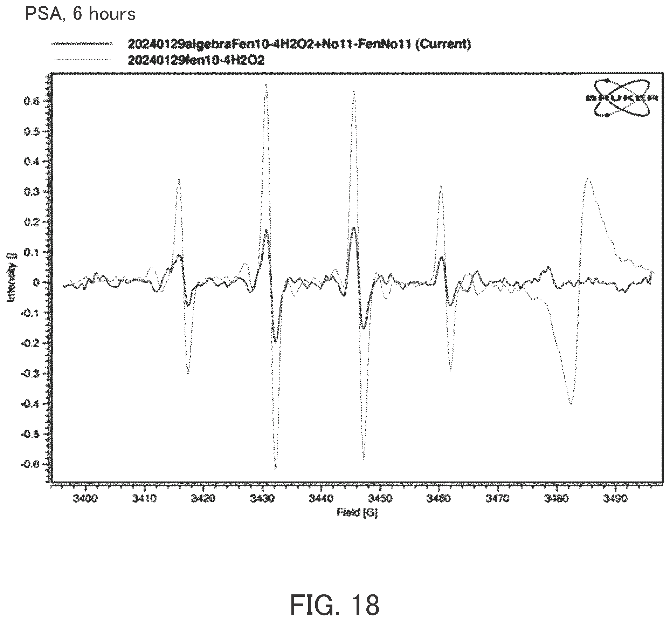

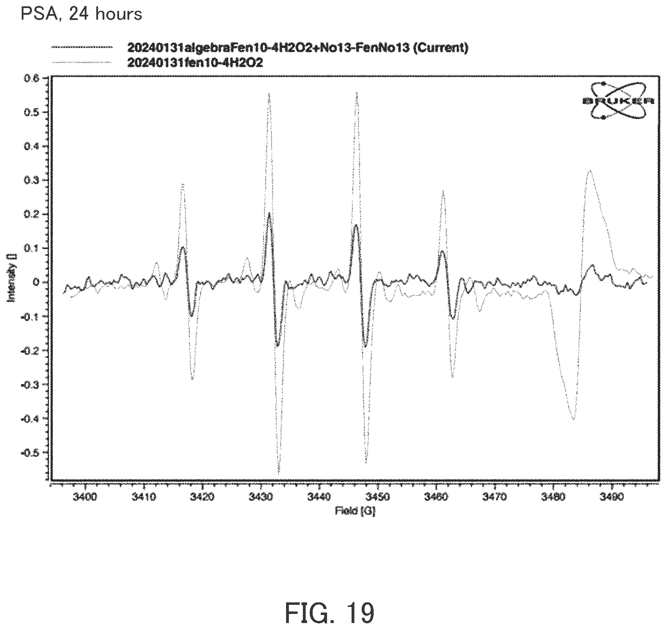

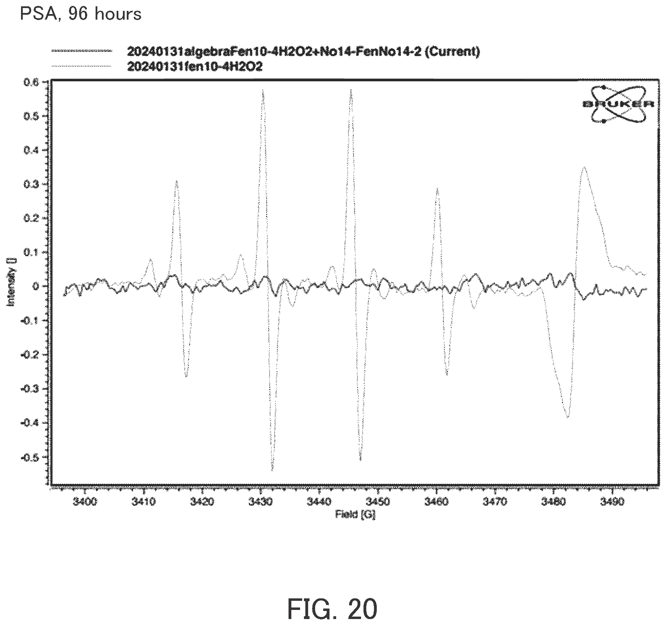

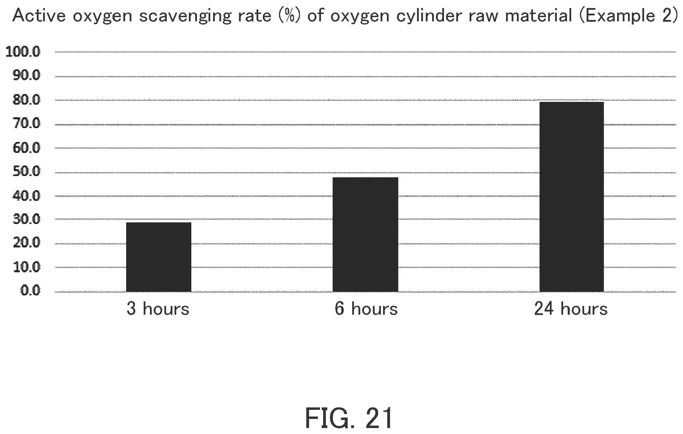

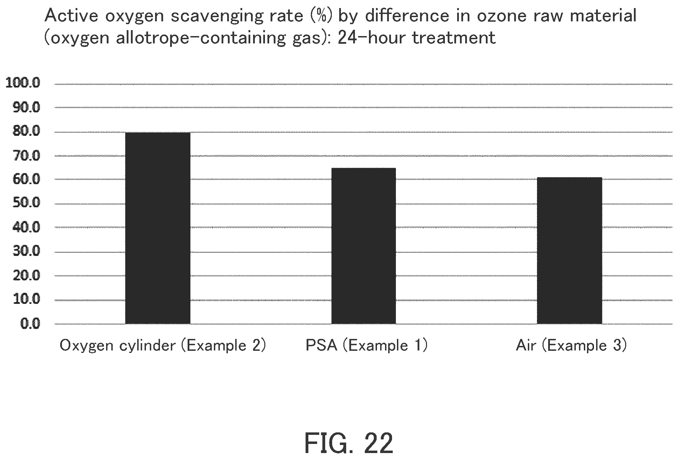

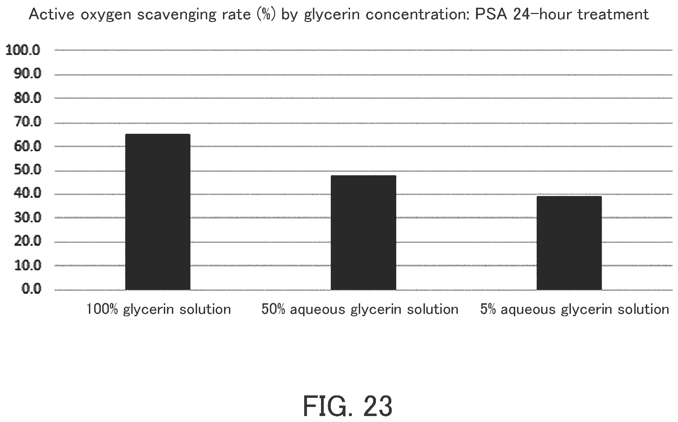

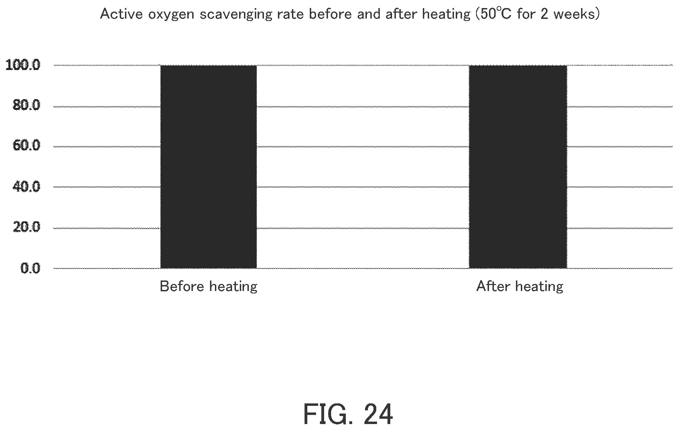

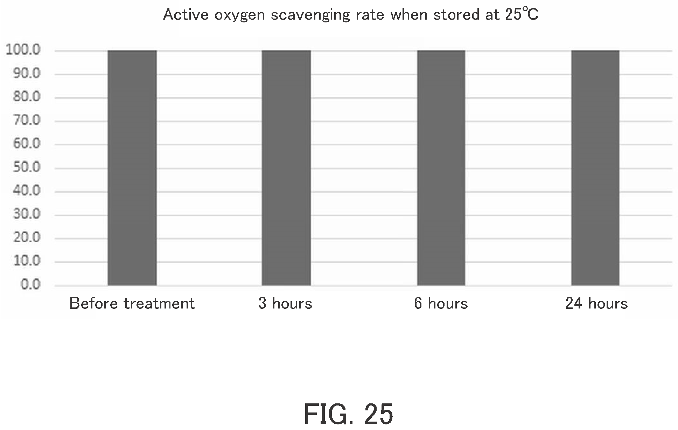

In Example 1, oxygen-treated glycerin solutions including oxygen-treated glycerin (active oxygen scavengers) were produced by changing the treatment time with ozone to 3 hours, 6 hours, 24 hours, and 96 hours, respectively. Next, in Examples 2 and 3, oxygen-treated glycerin solutions including oxygen-treated glycerin (active oxygen scavengers) were produced by changing the treatment time with an oxygen (O₂) cylinder or air to 3 hours, 6 hours, and 24 hours, respectively. For each of these, the active oxygen scavenging rate (active oxygen scavenging function) was verified by the method of (2) of Example 1 described above. FIGs. 11 to 13 show the ESR spectra of Example 2 treated with oxygen (O₂) in cases of treatment times of 3 hours, 6 hours, and 24 hours, respectively. FIGs. 14 to 16 show the ESR spectra of Example 3 treated with air in cases of treatment times of 3 hours, 6 hours, and 24 hours, respectively. FIGs. 17 to 20 show the ESR spectra of Example 1 ozone-treated with a PSA (oxygen concentrator) in cases of treatment times of 3 hours, 6 hours, 24 hours, and 96 hours, respectively. The active oxygen scavenging rates (%) calculated from these were 34.2%, 70.9%, 65.0%, and 100% for treatment times of 3 hours, 6 hours, 24 hours, and 96 hours, respectively, in the case of Example 1 ozone-treated with the PSA (oxygen concentrator). The active oxygen scavenging rates (%) in the cases of treatment times of 3 hours, 6 hours, and 24 hours of Example 2 treated with oxygen (O₂) were 28.8%, 48.1%, and 79.7%, respectively. The active oxygen scavenging rates (%) in the cases of treatment times of 3 hours, 6 hours, and 24 hours of Example 3 treated with air were 31.5%, 35.6%, and 60.8%, respectively. Note that these active oxygen scavenging rates (%) are relative numerical values with the active oxygen scavenging rate in the case of Example 1 ozone-treated with the PSA (oxygen concentrator) for 96 hours as 100%. The bar graph of FIG. 21 shows the active oxygen scavenging rates (%) of Example 2 ozone-treated using oxygen (O₂) as a raw material, in cases of treatment times of 3 hours, 6 hours, and 24 hours. As shown in FIG. 21, even if the ozone treatment time was short, a high active oxygen scavenging rate (%) was shown, and the longer the ozone treatment time was, the higher the active oxygen scavenging rate (%) was shown. In addition, the bar graph of FIG. 22 shows the active oxygen scavenging rates (%) of Examples 1 to 3 in a case of a treatment time of 24 hours, respectively. As shown in FIG. 22, a high active oxygen scavenging function was obtained regardless of whether an oxygen cylinder, a PSA, or air was used as a supply source of a raw material for ozone. Furthermore, in Example 1 ozone-treated with the PSA, glycerin was changed to a 50% aqueous glycerin solution and a 5% aqueous glycerin solution, and ozone treatment was performed respectively. FIG. 23 shows the active oxygen scavenging rates (%) of each of these in a case where the ozone treatment time was 24 hours. As shown in FIG. 23, the higher the glycerin concentration was, the higher the active oxygen scavenging rate (%) was shown, but even if the glycerin concentration was 5%, a sufficiently high active oxygen scavenging rate (%) was shown as compared with the untreated glycerin (see FIG. 12). In addition, regarding the case of the 96-hour treatment of Example 1 in which the highest active oxygen scavenging rate was obtained, the ozone-treated glycerin solution after the 96-hour treatment was further heated at 50°C for 2 weeks. As a result, as shown in the bar graph of FIG. 24, the active oxygen scavenging rate remained unchanged at 100% before and after heating. Accordingly, it could be verified that the ozone-treated glycerin having an active oxygen scavenging function included in the ozone-treated glycerin solution of the present Example is extremely stable even under a heating condition at 50°C. In addition, as shown in the bar graph of FIG. 25, the same result was obtained even when stored by being allowed to stand at room temperature for 3 hours, 6 hours, or 24 hours instead of heating at 50°C.

### [Comparison of pH of Examples 1 to 3]

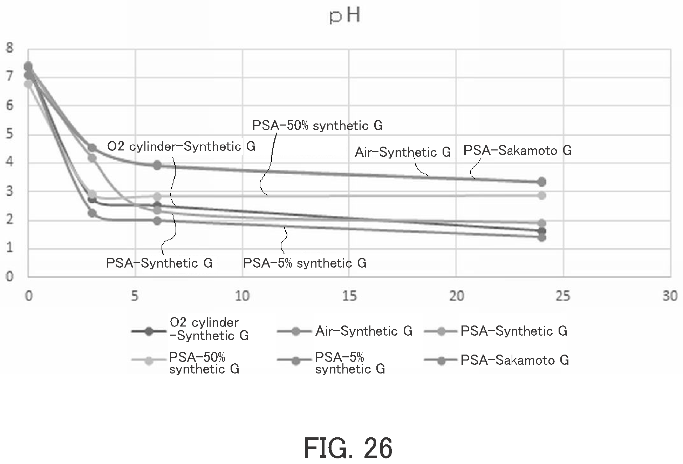

In each of Examples 1 to 3, the correlation between the treatment time and the pH is shown in the graph of FIG. 26. In FIG. 26, the horizontal axis is the treatment time, and the vertical axis is the pH. The pH was measured after each glycerin solution was diluted 10-fold with water. In addition, in FIG. 26, "O2 cylinder-synthetic G" represents Example 2 ozone-treated using oxygen in an oxygen (O₂) cylinder as a raw material. "Air-synthetic G" represents Example 3 ozone-treated using oxygen in air as a raw material. "PSA-synthetic G" represents Example 1 ozone-treated with a PSA (oxygen concentrator). "PSA-50% synthetic G" represents a case where synthetic glycerin was ozone-treated after being diluted to 50% with water in Example 1. "PSA-5% synthetic G" represents a case where synthetic glycerin was ozone-treated after being diluted to 5% with water in Example 1. "PSA-Sakamoto G" represents a case where synthetic glycerin was changed to glycerin manufactured by Sakamoto Yakuhin Kogyo Co., Ltd. in Example 1. As shown in FIG. 26, the pH was within the range of 1.00 to 5.00 in all cases.

While the present disclosure has been described above with reference to the embodiments and examples, the present disclosure is by no means limited thereto. Various changes and modifications that may become apparent to those skilled in the art may be made in the configuration and specifics of the present disclosure without departing from the scope of the invention of the present application.

Some or all of the above embodiments and examples may be described as in the following Supplementary Notes, but are not limited thereto.

### (Supplementary Note 1)

An active oxygen scavenger, including:
an ozone-treated alcohol ozone-treated with an oxygen allotrope-containing gas.

### (Supplementary Note 2)

The active oxygen scavenger according to Supplementary Note 1, wherein
the ozone-treated alcohol is ozone-treated glycerin.

### (Supplementary Note 3)

The active oxygen scavenger according to Supplementary Note 1 or 2, wherein
the ozone-treated alcohol includes an ozone-treated alcohol having an active oxygen scavenging function.

### (Supplementary Note 4)

An active oxygen scavenging method, including:
reacting an ozone-treated alcohol ozone-treated with an oxygen allotrope-containing gas with active oxygen.

### (Supplementary Note 5)

The active oxygen scavenging method according to Supplementary Note 4, wherein
the reacting is reacting the active oxygen scavenger according to any one of Supplementary Notes 1 to 3 with active oxygen.

### (Supplementary Note 6)

A method for producing an active oxygen scavenger, including:
ozone-treating an alcohol with an oxygen allotrope-containing gas.

### (Supplementary Note 7)

The method according to Supplementary Note 6, wherein
the alcohol is glycerin.

### Industrial Applicability

As described above, according to the present disclosure, a novel active oxygen scavenger, active oxygen scavenging method, and method for producing an active oxygen scavenger can be provided. Since the active oxygen scavenger of the present disclosure has an active oxygen scavenging function, it is extremely useful in fields of, for example, pharmaceuticals, cosmetic preparations, foods and drinks, and oral compositions.

This application claims priority from Japanese Patent Application No. 2024-023291 filed on February 19, 2024. The entire subject matters of the Japanese Patent Applications are incorporated herein by reference.

## Claims

1. An active oxygen scavenger, comprising:
an ozone-treated alcohol ozone-treated with an oxygen allotrope-containing gas.

2. The active oxygen scavenger according to claim 1, wherein
the ozone-treated alcohol is ozone-treated glycerin.

3. The active oxygen scavenger according to claim 1 or 2, wherein
the ozone-treated alcohol comprises an ozone-treated alcohol having an active oxygen scavenging function.

4. An active oxygen scavenging method, comprising:
reacting an ozone-treated alcohol ozone-treated with an oxygen allotrope-containing gas with active oxygen.

5. The active oxygen scavenging method according to claim 4, wherein
the reacting is reacting the active oxygen scavenger according to claim 1 with active oxygen.

6. A method for producing an active oxygen scavenger, comprising:
ozone-treating an alcohol with an oxygen allotrope-containing gas.

7. The method according to claim 6, wherein
the alcohol is glycerin.
